# EUROPÄISCHE PATENTANMELDUNG

(11) **EP 0 824 905 A1**
(43) Veröffentlichungstag der Anmeldung: **25.02.1998**
(21) Anmeldenummer: 97112473.0
(22) Anmeldetag: 21.07.1997
(51) Int. Cl.: A61F 5/453

(54) **Vorrichtung zur Ableitung von unkontrolliertem Harnabgang**

(30) Priorität: 21.08.1996 DE 19633605
(71) Anmelder: Tarob Consultants Ltd., St. Helier, Jersey JE4 8TQ (GB)
(72) Erfinder: Grundke, Reinhold, 84489 Burghausen (DE); Jaeniche, Wilhelm, 77694 Kehl-Leutesheim (DE); Lehmann, Harald, 89129 Langenau (DE)
(74) Vertreter: Hano, Christian, Dipl.-Ing.

(57) **Zusammenfassung**

Eine Vorrichtung zur Ableitung von unkontrolliertem Harnabgang bei männlichen Personen besitzt einen Grundkörper (10) aus weichem, schmiegsamem Material, dessen mittlerer Abschnitt einen etwa kreiszylinderischen, auf den Penisquerschnitt abgestimmten Mantel (12) bildet, an den sich am einen Ende eine sich trichterförmig in Richtung auf einen Schlauchanschluß (14) verengende Auffangkammer (13) anschließt. Am anderen, offenen Ende ist durch in Umfangsrichtung aufeinanderfolgende Unterbrechungen (18a, 18b, 18c) der Mantel (12) in mehrere Zungen (16a, 16b, 16c) unterteilt, wobei am Grundkörper (10) am Übergang vom geschlossenen Mantel (12) zu den Zungen (16a, 16b, 16c) eine weichelastische, schmiegsame Manschette (35) angebracht bzw. anbringbar ist, die geeignet ist, die Zungen von außen elastisch zu umschließen.

## Beschreibung

Die Erfindung betrifft eine Vorrichtung zur Ableitung von unkontrolliertem Harnabgang bei männlichen Personen.

Bei unkontrolliertem Harnabgang (Inkontinenz) wird nach dem heutigen Stand der Schulmedizin entweder ein Katheder in die Blase eingeführt, der den Harn in einen Auffangbehälter ableitet, oder es wird auf eine Ableitung verzichtet und die an Inkontinenz leidende Person gewindelt. Beide Verfahrensweisen sind mit Nachteilen behaftet, so setzt z.B. das Einführen eines Katheders Sachkunde voraus und ist mit Infektionsgefahr verbunden, während beim Windein eine Wäscheverschmutzung nicht ausgeschlossen werden kann. Zudem sind beide Verfahrensweisen relativ aufwendig, da durchschnittlich etwa sieben Windein zu jeweils etwa DM 3,-- pro Tag benötigt werden, bzw. der Katheder nach fünf, spätestens aber nach sieben Tagen gewechselt werden muß und dabei jeweils Kosten von etwa DM 28,-- anfallen.

Aufgabe der Erfindung ist es, eine Vorrichtung zu schaffen, die einfach zu handhaben und anzuwenden ist, eine Wäscheverschmutzung bei sachgemäßer Anwendung ausschließt, die Bewegungsfreiheit des Patienten so wenig wie möglich beeinträchtigt und zudem wiederholt verwendbar ist.

Die Lösung dieser Aufgabe dient eine Vorrichtung zur Ableitung von unkontrolliertem Harnabgang bei männlichen Personen mit einem Grundkörper aus verformbarem Material, der einen Ab-schnitt in Form eines etwa kreiszylinderischen, auf den Penisquerschnitt abgestimmten Mantels aufweist, an den sich am einen Ende eine sich trichterförmig in Richtung auf einen Schlauchanschluß verengende Auffangkammer anschließt, während sich am anderen, offenen Ende eine weichelastische, schmiegsame Haltemanschette anschießt.

Vor dem Ansetzen der Vorrichtung wird die Manschette auf den Mantel aufgerollt, worauf die Vorrichtung auf den Penis aufgeschoben und anschließend die Manschette abgerollt wird, um die Vorrichtung durch Hautkontakt zumindest der Zungen festzuhalten, die durch ihre Gestaltung aus weichelastischem, schmiegsamem Material auch beim Anschwellen des Penis keinen Blutstau hervorrufen können. Anschließend wird die Schlauchverbindung zwischen dem Schlauchanschluß und einem Auffangbeutel hergestellt. Durch die trichterförmige Gestaltung der Auf-fangkammer wird eine Rückwirbelung bei Harnabgang verhindert. Durch die Zungen ist sichergestellt, daß nach dem Aufschieben der Vorrichtung auf den Penis der zum Ausrollen der Manschette erforderliche Platz zur Verfügung steht.

Die Vorrichtung kann nicht nur im Liegen, sondern auch beim Stehen, Gehen und Sitzen getragen werden.

Eine besonders zweckmäßige Ausführungsform besteht darin, daß einerseits am Grundkörper am Übergang vom geschlossenen Mantel zu den Zungen und andererseits am Ende der Zungen jeweils ein Paar von in Umfangsrichtung verlaufenden und zwischen sich eine nach außen offene Rinne einschließende Wulste angeformt sind und daß eine elastische, schmiegsame Manschette an ihren offenen Enden jeweils mit einem umlaufenden, zum Eingriff in jeweils eine dieser Rinnen geeigneten Wulst versehen ist. Diese Konstruktion ermöglicht auf einfache Weise einen Austausch der Manschette.

Vorzugsweise besteht der Grundkörper aus Weichplastik und die Manschette aus Latex.

Eine zweckmäßige Ausgestaltung ist es, daß sich der Schlauchanschluß in Achsrichtung an die trichterförmige Auffangkammer anschließt.

Vorzugsweise ist am Mantel eine Anschlußstelle für eine Spülvorrichtung vorgesehen, wobei nach einer vorteilhaften Ausgestaltung die Anschlußstelle als geteilte Membran ausgebildet ist, die für den Anschluß des Konus einer Einmalspritze geeignet ist.

Nach einer weiteren zweckmäßigen Ausführungsform weist der Mantel nahe dem Übergang zu den Zungen eine Belüftungsöffnung auf.

Ein andere vorteilhafte Ausgestaltung ist es, daß die Zungen mit Stanzaussparungen zur Verbesserung der Hauthaftung versehen sind.

Anhand der nun folgenden Beschreibung eines in der Zeichnung dargestellten Ausführungsbeispiels der Erfindung wird diese näher erläutert.

Es zeigt:

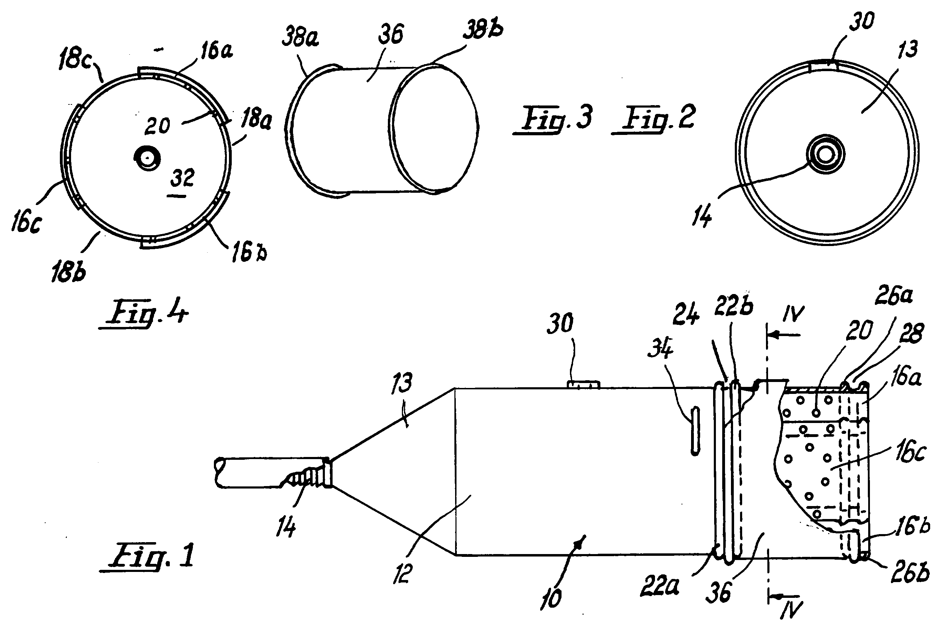

- Fig. 1: eine Seitenansicht der erfindungsgemäßen Vorrichtung,
- Fig.2: eine Ansicht auf die in Fig. 1 gezeigte Vorrichtung von links,
- Fig. 3: eine perspektivische Darstellung der Manschette und
- Fig. 4: einen Schnitt durch den Grundkörper nach der Linie IV-IV in Fig. 1.

Die gezeigte Vorrichtung besitzt einen Grundkörper 10 aus Weichplastik, der sich aus einem kreiszylinderischen Mantel 12 und einer sich an dessen einem Ende anschließenden, sich konisch verjüngenden Auffangkammer 13 mit einem an deren Ende angebrachten Schlauchanschlußstutzen 14 zusammensetzt. Das von der Auffangkammer 13 abgewandte Ende des Grundkörpers 10 läuft in drei in Umfangsrichtung mit gleichmäßigen Abständen aufeinanderfolgende Zungen 16a, 16b und 16c aus, die durch drei in Umfangsrichtung aufeinanderfolgende Unterbrechungen 18a, 18b und 18c voneinander getrennt sind, wobei der Zenrumswinkel der Zungen etwas größer ist als derjenige der Unterbrechungen. Die Zungen 16a bis 16c sind mit Stanzaussparungen 20 versehen, die dazu dienen, die Hauthaftung zu verbessern und ein Verrutschen der Vorrichtung zu verhindern.

Das den Zungen 16a bis 16c zugewandte Ende des Mantels 12 ist mit einem Paar umlaufender Wulste 22a und 22b versehen, die zwischen sich eine nach außen geöffnete Rinne 24 einschließen.

Ein ähnliches Paar von Wulsten 26a und 26b ist an den freien Enden der Zungen 16a bis 16c vorgesehen und schließt eine in Umfangsrichtung verlaufende, von den Unterbrechungen 16a bis 18c in drei Abschnitte unterteilte Rinne 28 ein.

Der Mantel 12 enthält eine geteilte Membran 30, die geeignet ist, das Ansetzen des Konus einer Einmalspritze zu ermöglichen, um eine Spülung des vom Mantel 12 umschlossenen Innenraums 32 durchzuführen.

Zur Belüftung dieses Innenraums ist eine schlitzförmige Öffnung 34 vorgesehen.

Eine Manschette 36 aus Latex ist an ihren beiden offenen Enden mit jeweils einem umlaufenden Wulst 38a bzw. 38b versehen und so dimensioniert, daß die Manschette 36 die Zungen 16a bis 16c in einer den Mantel 12 etwa koaxial fortsetzenden Position eng anliegend umschließen kann und dabei die Wulste 34a und 38b in die Rinnen 24 bzw. 28 eingreifen.

Vor der Benutzung wird die Manschette 36 mit ihrem den Wulst 38b aufweisenden Ende in Richtung auf den Mantel 12 zurückgerollt, wobei der Wulst 38a in der Rinne 24 verbleibt. Sodann wird die Vorrichtung auf den Penis aufgeschoben und positioniert, worauf die Manschette 36 abgerollt wird. bis der Wulst 38b in die Rinne 28 eingreift. Dadurch werden die Zungen 15a bis 16c gegen den Penis gelegt, um die Vorrichtung festzuhalten, wobei jedoch die Zungen ausweichen können, wenn der Penis anschwillt.

## Patentansprüche

1. Vorrichtung zur Ableitung von unkontrolliertem Harnabgang bei männlichen Personen mit einem Grundkörper (10) aus weichem, schmiegsamem Material, dessen mittlerer Abschnitt einen etwa kreiszylinderischen, auf den Penisquerschnitt abgestimmten Mantel (12) bildet, an den sich am einen Ende eine sich trichterförmig in Richtung auf einen Schlauchanschluß verengende Auffangkammer (13) anschließt, während am anderen, offenen Ende durch in Umfangsrichtung aufeinanderfolgende Unterbrechungen (18a, 18b, 18c) der Mantel (12) in mehrere Zungen (16a, 16b, 16c) unterteilt ist, wobei am Grundkörper (10) am Übergang vom geschlossenen Mantel (12) zu den Zungen (16a, 16b, 16c) eine weichelastische, schmiegsame Manschette (36) angebracht bzw. anbringbar ist, die geeignet ist, die Zungen (16a, 16b, 16c) von außen elastisch zu umschließen.

2. Vorrichtung nach Anspruch 1, *dadurch gekennzeichnet*, daß einerseits am Grundkörper (10) am Übergang vom geschlossenen Mantel (12) zu den Zungen (16a, 16b, 16c) und andererseits am Ende der Zungen jeweils ein Paar von in Umfangsrichtung verlaufenden und zwischen sich eine nach außen offene Rinne (24,; 28) einschließende Wulste (22a, 22b; 26a, 26b) angeformt sind, und daß die Manschette (36) an ihren offenen Enden jeweils einen umlaufenden, zum Eingriff in jeweils eine dieser Rinnen (24; 28) geeigneten Wulst (38a, 38b) aufweist.

3. Vorrichtung nach einem der Ansprüche 1 oder 2, *dadurch gekennzeichnet*, daß der Grundkörper (10) aus Weichplastik besteht.

4. Vorrichtung nach einem der vorhergehenden Ansprüche, *dadurch gekennzeichnet*, daß die Manschette (36) aus Latex besteht.

5. Vorrichtung nach einem der vorhergehenden Ansprüche, *dadurch gekennzeichnet*, daß sich der Schlauchanschluß (14) in Achsrichtung an die trichterförmige Auffangkammer (13) anschließt.

6. Vorrichtung nach einem der vorhergehenden Ansprüche, *dadurch gekennzeichnet*, daß am Mantel (12) eine Anschlußstelle für eine Spülvorrichtung vorgesehen ist.

7. Vorrichtung nach Anspruch 6, *dadurch gekennzeichnet*, daß die Anschlußstelle als geteilte Membran (30) ausgebildet ist, die für den Anschluß des Konus einer Einmalspritze geeignet ist.

8. Vorrichtung nach einem der vorhergehenden Ansprüche, *dadurch gekennzeichnet*, daß der Mantel (12) nahe dem Übergang zu den Zungen (16a, 16b, 16c) eine Belüftungsöffnung (34) aufweist

9. Vorrichtung nach einem der vorhergehenden Ansprüche, *dadurch gekennzeichnet*, daß die Zungen (16a, 16b, 16c) mit Stanzaussparungen (20) zur Verbesserung der Hauthaftung versehen sind.
